# EUROPEAN PATENT APPLICATION

(11) **EP 4 233 692 A1**
(43) Date of publication of application: **30.08.2023**
(21) Application number: 22178848.2
(22) Date of filing: 14.06.2022
(51) Int. Cl.: A61B 5/00, A61B 5/01, A61B 5/024, A61B 5/11

(54) **ELECTRONIC FINGER RING FOR MONITORING HEALTH AND FITNESS IN REAL TIME**

(30) Priority: 28.02.2022 IN 202241010714
(71) Applicant: Ultrahuman Healthcare Pvt Ltd, 560025 Bengaluru (Karnataka) (IN)
(72) Inventor: SHANKAR, Apoorv, 560102 Bangalore (IN); NAMDEO, Yogansh, 560100 Bengaluru (IN); JAYAN, Anoop, 560058 Bangalore (IN)
(74) Representative: Fioravanti, Corrado

(57) **Abstract**

An electronic finger ring (100) comprises an outer layer (202), a middle layer (204), and an inner layer (206). The outer layer (202) is made of a rigid and antirust material or coating, such as of titanium. The middle layer (204) is a flexible Printed Circuit Board (PCB) comprising one or more sensors (302, 304, 306) connected with a microcontroller (308) and a battery (312). The flexible PCB is encapsulated in a transparent resin layer i.e. the inner layer (206) or sandwiched between the inner layer (206) and the outer layer (202). The one or more sensors (302, 304, 306) capture values of one or more fitness and health parameters of a user for tracking his lifestyle, activities, and habits.

## Description

### FIELD OF INVENTION

The present invention relates to a system for health and fitness monitoring in real time through a finger wearable device/ring.

### BACKGROUND

Regular health and fitness monitoring is important for accomplishing long term health and wellness goals.

Traditionally, fitness monitoring required manual recording of one's physical exercise activities. However, such manual recording is inconvenient and often inaccurate.

Generally, detection of one's health parameters require stationary medical equipment such as an ECG machine, BP monitoring machine etc. Consequently, a person always on the move is unable to keep a track of his physical health parameters to maintain his health and good condition thereby disabling him to have more control on his daily lifestyle, physical activities and habits. Needless to say, lack of knowledge about one's own body and inability to make conscious data driven lifestyle changes impacts one's health and fitness in the long run.

Thus, there remains a need for a wearable device for tracking and analysing health and fitness through trends of various health and metabolic parameters thereby enabling users to optimise their food intake, workout routines, sleep and other general daily lifestyle activities.

### OBJECTS OF THE INVENTION

A general objective of the present invention is to allow a wearer/user to conduct real time monitoring of health and fitness parameters.

Another objective of the present invention is to provide a cost-effective mechanism for measurement of health and fitness parameters through a device that is convenient to use on a regular basis

Yet another objective of the present invention is to provide a mechanism for measurement of health and fitness parameters that could be assembled quickly and upgraded easily.

### SUMMARY OF THE INVENTION

The summary is provided to introduce aspects related to an electronic finger ring for monitoring fitness and health parameters of users. The electronic finger ring may include different sensors for monitoring the fitness and health parameters of users.

In one aspect, the electronic finger ring may comprise an outer layer, a middle layer, and an inner layer. The outer layer may be made of a rigid and antirust material or coating, such as of titanium. For use as the outer layer, a titanium shell may be manufactured using an injection moulding process or metal cutting process like Computer Numerical Control (CNC).

In one aspect, the middle layer positioned between the outer layer and the inner layer may be a flexible Printed Circuit Board (PCB). The flexible PCB may house one or more sensors to capture fitness and health parameters of a user.

In one aspect, the inner layer may be made by solidification of a resin poured in a liquid form in cavity of the outer layer after placement of the middle layer in the cavity. The inner layer may be made of a translucent, or completely transparent material. Materials such as glass, plastic, resin, silicone, or epoxy based material may be used to fabricate the inner layer. Transparency of the inner layer, for visible and near visible light, would allow the sensors to obtain reading from the finger of the user.

In one aspect, wherein the inner layer may be made of one or more detachable parts configured to snap together and attach with the outer layer.

In one aspect, the inner layer may be assembled over the outer layer through one or more of snap fitting, tight fitting, gushing screws, and magnets.

In one aspect, a microcontroller may be mounted on the flexible PCB. All the sensors mounted on the flexible PCB may be connected to the microcontroller. The sensors may transmit values of the fitness and health parameters detected by them to the microcontroller, in real-time. The microcontroller may obtain values of the fitness and health parameters from the sensors based on some internal and external triggers associated with the sensors. The microcontroller may also store values of the fitness and health parameters in its own memory or a separate memory element mounted on the flexible PCB.

In one aspect, a wireless module may also be mounted on the flexible PCB to wirelessly communicate the fitness and health parameters and the secondary parameters to an external device, such as a smartphone or a laptop. The wireless module may work on one or more of Bluetooth and Near Field Communication (NFC).

In one aspect, a battery may be used to power the sensors used in the electronic finger ring.

In one aspect, a wireless charging coil may be connected with the battery and positioned in vicinity of the middle layer to enable wireless charging of the battery.

In one aspect, the electronic ring may be configured to be worn on a finger of the user.

In one aspect, a window may be provided in the outer layer to house a NFC transceiver for communication with external devices.

In another aspect, an electronic ring for monitoring fitness and health parameters of a user may comprise an outer layer made of a rigid material, multiple detachable concentric layers stacked over each other. One or more layer of the multiple detachable concentric layers may include one or more electronic components for capturing fitness and health parameters of the user. An innermost layer of the multiple concentric layers may be made up of a transparent or translucent material. The innermost layer of the multiple concentric layers may be configured to be in contact with skin of the user.

In one aspect, the one or more electronic components may include a microcontroller, one or more sensors, a wireless module, and a battery to power the one or more sensors.

In one aspect, the multiple concentric layers may be attached with each other using one or more of mechanical fitting methods, magnetic coupling, and using adhesives.

Other aspects and advantages of the invention will become apparent from the following description, taken in conjunction with the accompanying drawings, illustrating by way of example the principles of the invention.

### BRIEF DESCRIPTION OF THE DRAWINGS

The accompanying drawings constitute a part of the description and are used to provide further understanding of the present invention. Such accompanying drawings illustrate the embodiments of the present invention which are used to describe the principles of the present invention. The embodiments are illustrated by way of example and not by way of limitation in the figures of the accompanying drawings in which like references indicate similar elements.

It should be noted that references to "an" or "one" embodiment in this invention are not necessarily to the same embodiment, and they mean at least one. In the drawings:
Fig. 1 illustrates a front perspective view of an electronic finger ring, in accordance with an embodiment of the present invention;
Fig. 2 illustrates an exploded view of the electronic finger ring, in accordance with an embodiment of the present invention;
Fig. 3 illustrates a side view of a middle layer/flexible PCB used in the electronic finger ring, in accordance with an embodiment of the present invention;
Fig. 4 illustrates a side perspective view of an electronic ring including a window, in accordance with an embodiment of the present invention;
Figs. 5a and 5b illustrate stages of a fabrication process of the electronic finger ring, in accordance with an embodiment of the present invention;
Figs. 6a and 6b illustrate stages of another fabrication process of the electronic finger ring, in accordance with an embodiment of the present invention;
Fig. 7 illustrates a modular electronic ring, in accordance with an embodiment of the present invention;
Figs. 8a and 8b illustrate top view and side view respectively of the electronic ring placed on a wireless charger , in accordance with an embodiment of the present invention; and
Fig. 9 illustrates an adjustable electronic ring, in accordance with an embodiment of the present invention.

### DETAILED DESCRIPTION OF THE INVENTION

The detailed description set forth below in connection with the appended drawings is intended as a description of various embodiments of the present invention and is not intended to represent the only embodiments in which the present invention may be practiced. Each embodiment described in this disclosure is provided merely as an example or illustration of the present invention, and should not necessarily be construed as preferred or advantageous over other embodiments. The detailed description includes specific details for the purpose of providing a thorough understanding of the present invention. However, it will be apparent to those skilled in the art that the present invention may be practiced without these specific details.

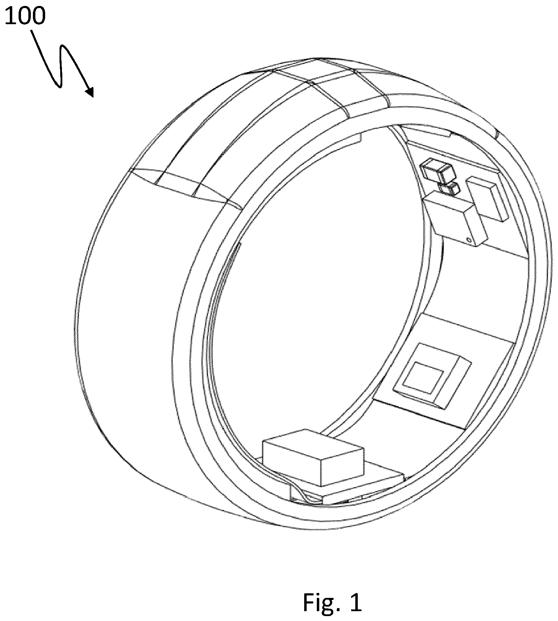

The proposed invention relates to an electronic ring (100) for monitoring fitness and health parameters of users. The electronic ring (100) may be worn by a user over a finger. Fig. 1 illustrates a front perspective view of the electronic ring (100), in accordance with an embodiment of the present invention. The electronic ring (100) may be developed in different sizes to fit over different fingers of different users. The electronic ring (100) may include different sensors for monitoring the fitness and health parameters of users. Types, placement, and working of the sensors used in the electronic ring (100) are described henceforth.

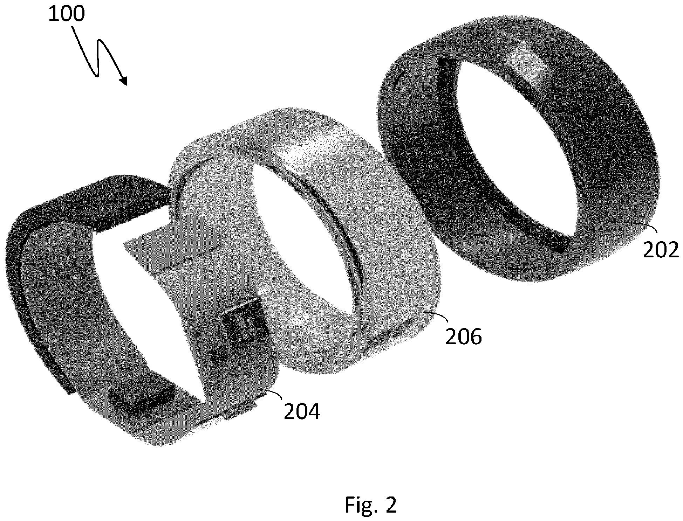

Fig. 2 illustrates an exploded view of the electronic ring (100), in accordance with an embodiment of the present invention. The electronic ring (100) may comprise an outer layer (202), a middle layer (204), and an inner layer (206). The outer layer (202) may be made of a rigid and antirust material, such as titanium. For use as the outer layer (202), a titanium shell may be manufactured using an injection moulding process. During the injection moulding process, titanium present in a powdered form may be reacted with oxides to obtain a mixture. The mixture may be melted and poured into a mould for obtaining the titanium shell. Alternatively, Computer Numerical Control (CNC) may be employed to and liquid metal or cut metal could be used to fabricate the outer layer (202).

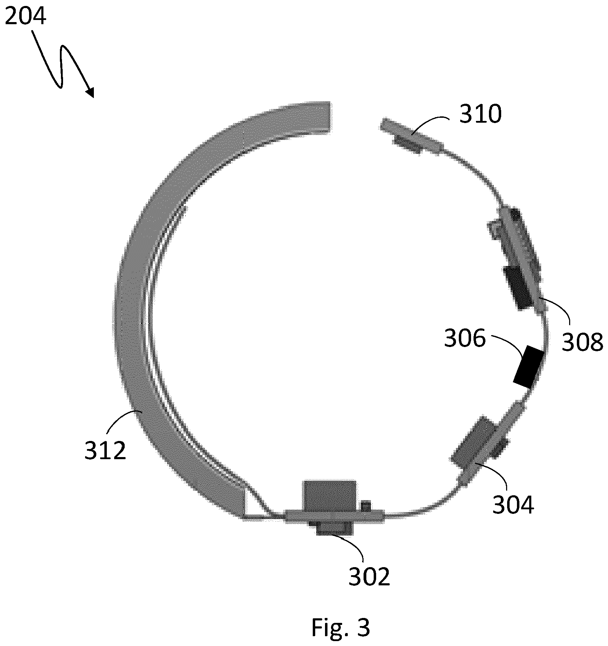

The middle layer (204) positioned between the outer layer (202) and the inner layer (206) may be a flexible Printed Circuit Board (PCB) or a rigid-flexible PCB. Although the description henceforth is provided considering a flexible PCB is used as the middle layer (204); however, in different implementations, flexibility or rigidity of the PCB may be modified as per requirement. Fig. 3 illustrates a side view of the middle layer i.e. the flexible PCB (204), in accordance with an embodiment of the present invention. The flexible PCB (204) may house one or more sensors (302, 304, 306) to capture fitness and health parameters of a user.

A microcontroller (308) may be mounted on the flexible PCB (204). All the sensors mounted on the flexible PCB (204) may be connected to the microcontroller (308). The sensors may transmit values of the fitness and health parameters detected by them to the microcontroller (308), in a real-time. The microcontroller (308) may obtain values of the fitness and health parameters from the sensors (302, 304, 306) based on some internal and external triggers associated with the sensors (302, 304, 306). The microcontroller (308) may also store values of the fitness and health parameters in its own memory or a separate memory element mounted on the flexible PCB (204).

The microcontroller (308) may analyse and process values of the fitness and health parameters received from the sensors (302, 304, 306) to determine secondary parameters.

A wireless module (310) may also be mounted on the flexible PCB (204) to wirelessly communicate the fitness and health parameters and the secondary parameters to an external device, such as a smartphone or a laptop. The wireless module (310) may work on one or more of Bluetooth and Near Field Communication (NFC).

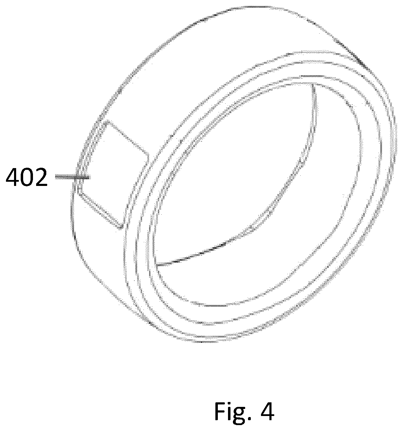

In one implementation, to enable NFC or short range transmission of signals from the electronic ring (100), a window may be provided in the outer layer (202) to house an NFC transceiver. Fig. 4 illustrates a side perspective view of the electronic ring (100) including a window, in accordance with an embodiment of the present invention. As illustrated in Fig. 4, the outer layer (202) may be provided with a window (402). The window (402) may be a slot provided in the outer layer (402) or be made of non-metallic material. The NFC transceiver housed in the window may enable applications like NFC payments, and ID card detection through the electronic ring (100). The NFC transceiver may communicate with other NFC devices that may come in close proximity of the electronic ring (100) when the user's hand is placed near such NFC devices.

Referring back to Fig. 3, a battery (312) may be used to power the sensors (302, 304, 306), the micro-controller (308), radio based wireless module (310), and any other sensor used in the electronic finger ring (100).

Referring again to Fig. 2, the inner layer (206) of the electronic finger ring (100) may come in contact of the user's finger once the user wears the electronic finger ring (100). A portion of or entire inner layer (206) may be made of a semi-transparent, translucent, or completely transparent material. Materials such as glass, plastic, resin, silicone, or epoxy based material may be used to fabricate the inner layer (206). Transparency of the inner layer (206), for visible and near visible light, would allow the sensors to obtain reading from the finger of the user. For example, an optical sensor may be able to transmit light and obtain reflection of the light through the inner layer (206).

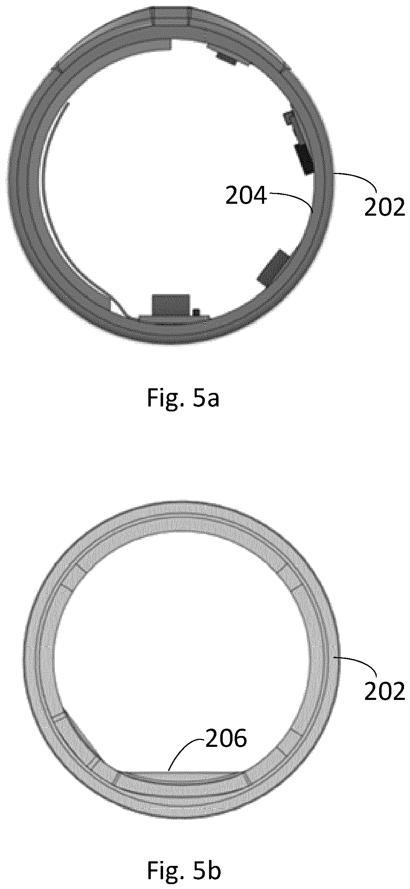

During fabrication of the electronic ring (100), different layers of the electronic ring (100) may be integrated in a stepwise manner. In one implementation, the outer layer (202) may be held in a ring shaped mould. The middle layer (flexible PCB) (204) may then be positioned close to the outer layer (202), as illustrated in Fig. 5a. Placement of the outer layer (202) and the middle layer (204) may create a gap between them. Successively, one or more plastic based resins present in a liquid form may be poured in the gap. The resins may also be poured to fill up any gaps in the flexible PCB (204). After solidification, a semi-transparent solid body of resins may get formed. The semi-transparent solid body of resins may encapsulate the flexible PCB (204) and keep it in contact with the outer layer (202). The semi-transparent solid body of resins settling beneath the flexible PCB (204) may form the inner layer (206) which comes in contact of the user's skin when the user wears the electronic finger ring (100). Transparency of the electronic finger ring (100) enables refraction of visible and IR light transmitted or received by one or more sensors used in the electronic ring (100). Through such process, the resin encapsulating flexible PCB (302) may be present in contact with the titanium shell, as illustrated in Fig. 5b. In this manner, the electronic ring (100) may be obtained.

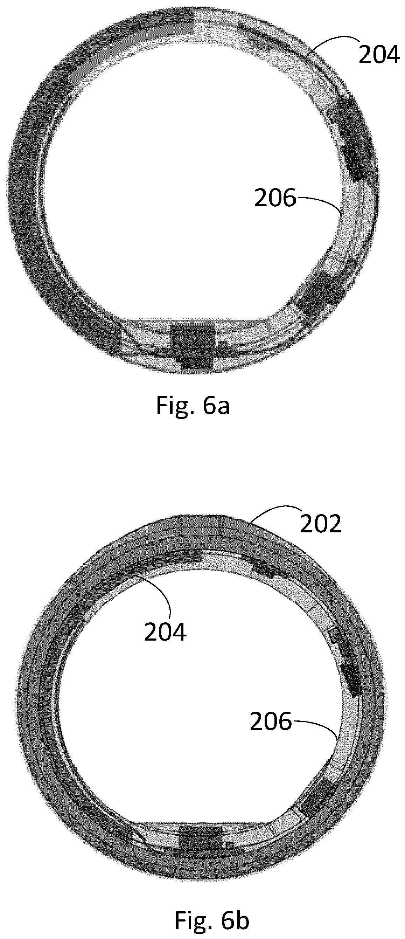

In an alternate implementation, the middle layer (flexible PCB) (204) may be covered using one or more plastic based resins present in a liquid form. After solidification of the resins, a semi-transparent solid body of resins may get formed. The semi-transparent solid body of resins may encapsulate the flexible PCB (204), as illustrated in Fig. 6a. The semi-transparent solid body of resins may encapsulate the flexible PCB (204) within a cavity present beneath the outer layer (202), to form the electronic ring (100), as illustrated in Fig. 6b.

In another implementation, the inner layer (206) may be made of two different transparent plastic based parts which may snap together to form a single piece which may be attached with the outer layer (202). Together, the two different transparent plastic based parts may form the inner layer (206) which comes in contact with skin of a user's finger.

Upon fixing the inner layer (206) with the outer layer (202), open gaps or cavities may be filled using adhesives or resins. In certain situations, gaps and cavities may get created in the inner layer (206) around the areas where certain sensors, such as light and temperature based sensors need complete transparency. Such gaps or cavities may be filled with Finite Infrared (FIR) and visible light-transparent epoxy based resin such that sensor readings do not get affected by non-transparency or other properties of material of the inner layer (206). Transparency enables the light and temperature based sensors such as heart rate sensor, motion sensor, and temperature sensor to record accurate readings by enabling reception and transmission of visible light or Infrared without any hinderance due to solid bodies in between the sensor and the user's skin.

In one implementation, to fabricate the electronic finger ring (100), the flexible PCB (204) may first be encapsulated in centre using resin. Successively, an outer section of the resin may be fitted into a metal shell acting as a durable outer cover of the electronic finger ring (100). The metal shell may be assembled over a solid form of resin through mechanical fitting methods, such as snap fitting, tight fitting, and using screws. Alternatively, the metal shell may be assembled over the resin using magnets. Glue may also be used to fix the metal shell on the resin. The metal shell may be made of multiple colours and textures such that it can be replaced as per the user's choice.

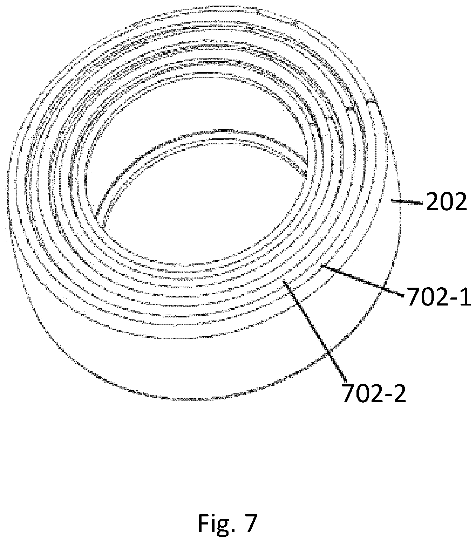

In another implementation, the electronic ring (100) may have a modular structure made up of multiple concentric layers Fig. 7 illustrates a modular electronic ring (100), in accordance with an embodiment of the present invention. The electronic ring (100) may comprise multiple concentric layers (702-n). Multiple concentric layers (702-n) may be positioned beneath the outer layer (202). Each of the multiple concentric layer (702-n) may be stacked over another concentric layer (702-n). An innermost layer of the multiple concentric layers (702-n) may be made up of a transparent or translucent material and is configured to be in contact with skin of the user. One or more layer of the multiple concentric layers (702-n) may include one or more electronic components such as sensors (302, 304, 306), battery (312), wireless module (310), and microcontroller (308). Different layers may be attached or detached with each other by mechanical fitting methods, magnetic coupling, or using adhesives. After attachment of all the multiple concentric layers (702-n)., electrical connections between each layer may be made using pogo pins, connector pads, wireless charging, or Near Field Communication (NFC) or Radio Frequency (RF) coils. Detachability of the multiple concentric layers (702-n) in the electronic ring (100) may enable the user to update the electronic ring (100) with new set of features by replacing one or more multiple concentric layers (702-n). To update the electronic ring (100), a user may be required to purchase only one layer of multiple concentric layers (702-n) having an updated feature, while other layers may be used without replacement. In this manner, cost of buying a whole new electronic ring (100) after every few years could be avoided. Additionally, the multiple concentric layers (702-n) may increase usable life of the electronic ring (100) by allowing easy replacement of a layer including the battery (312) as one of the main parts of any electronic device that get damaged or reach end of its usable life is a battery.

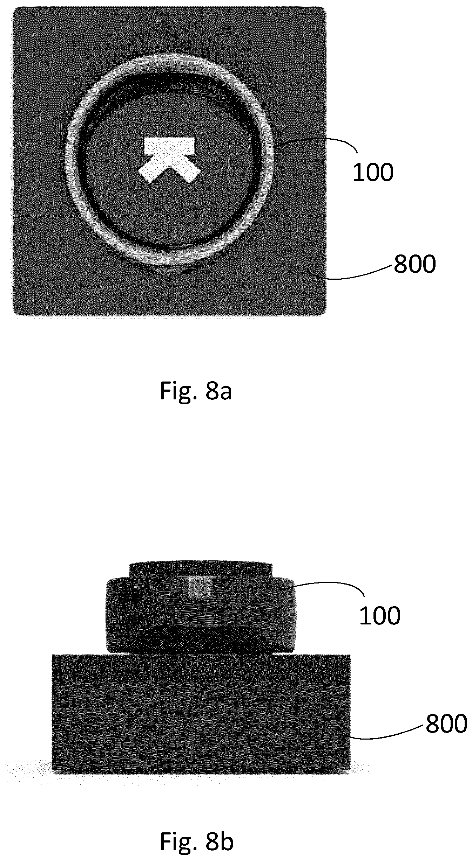

Figs. 8a and 8b illustrate top view and side view respectively of the electronic finger ring (100) placed on a wireless charger (800), in accordance with an embodiment of the present invention.

The electronic ring (100) may be wirelessly chargeable using the wireless charger (600). For wireless charging, the electronic ring (100) may comprise a wireless charging coil. The wireless charging coil may be positioned above or below the flexible PCB (204). Alternatively, the wireless coil may be a part of the flexible PCB (204) and may reside in the middle layer (204). Electromagnetic field generated by a coil present in the wireless charger (600) may get coupled with the wireless charging coil of the electronic finger ring (100) when the electronic finger ring (100) is present above the wireless charger (800). Through coupling of the electromagnetic field, power may be received and stored in the battery (312). The wireless charger (800) may itself include a battery of capacity sufficient to charge the battery (312) of the electronic finger ring (100) a few times.

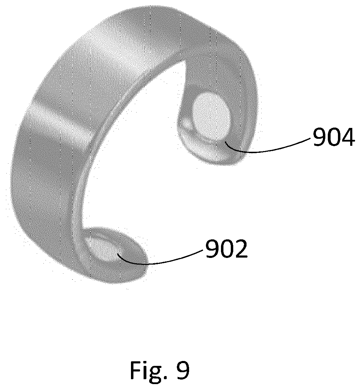

Fig. 9 illustrates an adjustable electronic ring, in accordance with an embodiment of the present invention. As illustrated in Fig. 9, a gap may be provided at two ends (902, 904) of the electronic ring (100) to enable the user to adjust or fit the electronic ring (100) in any finger.

The electronic finger ring (100) may be worn by a user at all times so that his fitness and health parameters are continuously tracked and reported to him. The electronic finger ring (100) provides a cost effective mean to non-invasively measure fitness and health parameters of a user in real time. With the data obtained from the electronic finger ring (100), a user may be able to track changes in his lifestyle, activities, and habits.

In the above detailed description, reference is made to the accompanying drawings that form a part thereof, and illustrate the best mode presently contemplated for carrying out the invention. However, such description should not be considered as any limitation of scope of the present unit. The structure thus conceived in the present description is susceptible of numerous modifications and variations, all the details may furthermore be replaced with elements having technical equivalence.

## Claims

1. An electronic ring (100) for monitoring fitness and health parameters of a user, the electronic ring (100) comprising:
an outer layer (202) made of a rigid material;
a middle layer (204) including a Printed Circuit Board (PCB) housing one or more sensors (302, 304, 306) for capturing fitness and health parameters of the user and a battery (312) to power the one or more sensors (302, 304, 306); and
an inner layer (206), at least a portion of which is made up of a transparent or translucent material, wherein the inner layer (206) is configured to be in contact with skin of the user.

2. The electronic ring (100) as claimed in claim 1, wherein the inner layer (206) is made of one or more detachable parts configured to snap together and attach with the outer layer (202).

3. The electronic ring (100) as claimed in claim 1, wherein the inner layer (206) is assembled over the outer layer (202) through one or more of snap fitting, tight fitting, gushing screws, adhesive, and magnets.

4. The electronic ring (100) as claimed in claim 1, wherein the inner layer (206) is made by solidification of a resin poured in a liquid form in a cavity of the outer layer (202) after placement of the middle layer (204) in the cavity.

5. The electronic ring (100) as claimed in claim 1, wherein the one or more sensors (302, 304, 306) remain in contact of at least one of the inner layer (202) and the outer layer (206) for accurately capturing readings of the fitness and health parameters.

6. The electronic ring (100) as claimed in claim 4, wherein the resin is a Finite Infrared (FIR) and visible light-transparent epoxy.

7. The electronic ring (100) as claimed in claim 1, wherein the outer layer (202) is shell shaped.

8. The electronic ring (100) as claimed in claim 1, wherein a microcontroller (308) and a memory are mounted on the PCB, wherein the microcontroller (308) is configured to obtain values of the fitness and health parameters detected by the one or more sensors including a heart rate sensor, a temperature sensor, a light sensor, an NFC transducer, and a motion sensor, in real-time and store the values in the memory.

9. The electronic ring (100) as claimed in claim 1, wherein the electronic finger ring (100) includes a wireless module (310) mounted on the PCB to wirelessly communicate the fitness and health parameters to an external device.

10. The electronic ring (100) as claimed in claim 1, wherein a wireless charging coil is positioned in vicinity of the middle layer (204) and connected with the battery (312) to enable wireless charging of the battery (312).

11. The electronic ring (100) as claimed in claim 1, wherein the electronic ring (100) is configured to be worn on a finger of a user.

12. The electronic ring (100) as claimed in claim 11, wherein a gap is present between two ends (902, 904) of the electronic ring (100) to enable adjustment of the electronic ring (100) over the finger of the user.

13. The electronic ring (100) as claimed in claim 1, wherein a window is provided in the outer layer (202) to house an NFC transceiver for communication with external devices.

14. An electronic ring (100) for monitoring fitness and health parameters of a user, the electronic ring (100) comprising:
an outer layer (202) made of a rigid material; and
multiple concentric layers (702-n) stacked over each other, wherein one or more layer of the multiple detachable concentric layers (702-n) includes one or more electronic components for capturing fitness and health parameters of the user,
wherein an innermost layer of the multiple concentric layer is made up of a transparent or translucent material and is configured to be in contact with skin of the user.

15. The electronic ring (100) as claimed in claim 14, wherein the one or more electronic components include a microcontroller (308), an NFC transducer, a wireless module (310), a heart rate sensor, a temperature sensor, a light sensor, a motion sensor, and a battery (312) to power the one or more one or more electronic components.

16. The electronic ring (100) as claimed in claim 14, wherein the multiple concentric layers (702-n) are attached with each other using one or more of mechanical fitting methods, magnetic coupling, and using adhesives.
